# EUROPÄISCHE PATENTSCHRIFT

(11) **EP 3 100 681 B1**
(45) Veröffentlichungstag und Bekanntmachung des Hinweises auf die Patenterteilung: **11.11.2020**
(21) Anmeldenummer: 16168930.2
(22) Anmeldetag: 10.05.2016
(51) Int. Cl.: A61B 6/10

(54) **STRAHLENSCHUTZANORDNUNG**
RADIATION PROTECTION ASSEMBLY
SYSTEME DE PROTECTION CONTRE LES RAYONNEMENTS

(30) Priorität: 12.05.2015 DE 102015208829
(43) Veröffentlichungstag der Anmeldung: 07.12.2016
(73) Patentinhaber: Mavig GmbH, 81829 München (DE)
(72) Erfinder: BUCHMEYER, Markus, 81829 München (DE)
(74) Vertreter: Vossius & Partner Patentanwälte Rechtsanwälte mbB

(56) Entgegenhaltungen:
- WO-A2-2009/124094
- WO-A2-2014/167544
- DE-A1-102012 218 391
- GB-A- 2 461 569
- US-A- 5 006 718
- US-A1- 2014 048 730

## Beschreibung

Die Erfindung betrifft eine Strahlenschutzanordnung insbesondere zur Anbringung an einer Trägerschiene, die an einer Seite eines Behandlungstisches angebracht ist. Die vorliegende Strahlenschutzanordnung ist insbesondere zur Verwendung in der interventionellen Radiologie geeignet und dient bei Operationen zum Schutz von beteiligtem Personal wie dem Arzt oder Assistenten gegen auftretende Strahlung, insbesondere Röntgenstrahlung.

Die DE 10 2012 218 391 A1 betrifft eine Strahlenschutzanordnung insbesondere zur Anbringung an einer Trägerschiene, die an der Seite eines Behandlungstisches angebracht ist. Die Strahlenschutzanordnung weist dabei mindestens einen schwenkbar gelagerten Haltegriff und einen Schließmechanismus auf, wobei der Schließmechanismus geeignet ist zum Verriegeln des Halters an der Trägerschiene und wobei der Schließmechanismus durch eine Schwenkbewegung des mindestens einen an dem Halter schwenkbar gelagerten Haltegriffs betätigbar ist.

Die DE 10 2009 025 380 A1 betrifft eine Strahlenschutzanordnung insbesondere zur Anbringung an einer Trägerschiene, die an der Seite eines Behandlungstisches angebracht ist. Die Strahlenschutzanordnung weist mindestens eine Lamelle aus einem Strahlenschutzmaterial und eine zugehörige Befestigungsvorrichtung auf. Die Lamelle ist an der Befestigungsvorrichtung schwenkbar befestigt. Die Befestigungsvorrichtung weist einen Halter auf, der auf die Trägerschiene aufsetzbar ist und mit einer Feststelleinrichtung ist der Halter an der Trägerschiene fixierbar.

Die WO 2004/107979 betrifft eine Strahlenschutzanordnung, die seitlich an einem medizinischen Untersuchungs- oder Behandlungstisch als Unterkörperschutz befestigbar ist. In einer Ausführungsform weist diese Strahlenschutzanordnung mehrere nebeneinander angeordnete Lamellen auf, die an einem Ende an einem gemeinsamen Trägerelement befestigt sind. Zusätzlich ist ein Oberteil vorgesehen, das an der Trägerschiene an dem Behandlungstisch befestigbar ist. Die Lamellen können bei dieser bekannten Strahlenschutzanordnung aus einer Bleigummimatte bestehen, die beispielsweise in PVC eingeschichtete Bleifolien aufweisen. Die Bleigummimatten werden in Umhüllungen eingesetzt, die aus einem einfach zu reinigenden und zu sterilisierenden Stoff bestehen. Die Lamellen sind in ihrer Länge veränderbar, in dem ein unteres Ende nach oben umgeschlagen wird und dieses Ende in dieser umgeschlagenen Position fixiert wird, beispielsweise mittels Druckknöpfen, Klettverschluss oder durch die Verwendung von Gurten.

Die DE 1 516 420 beschreibt eine Strahlenschutzvorrichtung mit mehreren einzelnen Bleigummilappen, die schwenkbar gelagert sind. Jeder Bleigummilappen ist um eine zu ihm senkrecht stehende, oberhalb seines Schwerpunktes befindliche Achse schwenkbar gelagert. In einer Ausführungsform ist ein kammartiger Träger vorhanden, bei dem an den Fortsätzen Zapfen vorhanden sind, an denen die einzelnen Bleigummilappen schwenkbar befestigt sind.

Die DE 1 466 848 beschreibt eine Strahlenschutzvorrichtung, bei der Bleigummilappen über Drehachsen an der Seitenkante eines Leuchtschirmträgers angebracht sind.

In der DE 27 49 826 A1 wird eine Röntgenstrahl-Abschirmeinrichtung beschrieben, bei denen Bleigummilappen vorhanden sind, die mit Klemmen um Achsen schwenkbar angebracht sind. In einer Ausführungsform weist eine Laufschiene ein T-förmiges Innenprofil auf, in das Halter einsetzbar sind.

Die DE 30 12 463 C2 zeigt eine Strahlenschutzvorrichtung, bei der Bleigummilappen schwenkbar an einem Träger aufgehängt sind. Bei einer Ausführungsform weist ein Tragelement kammartige Fortsätze auf, an denen die Bleigummilappen gelenkig gelagert sind.

Die DE 297 04 613 U1 beschreibt eine Strahlenschutzvorrichtung, bei der einzelne Bleigummilappen verschwenkbar an einer Schiene angeordnet werden können. In einer Ausführungsform ist für jeden Bleigummilappen ein Träger vorhanden, der in einer Führungsschiene verstellbar gelagert ist. Die Bleigummilappen sind an einem Träger um eine Querachse schwenkbar gelagert. Weiterhin sind Bremsmittel vorgesehen, mit denen verhindert wird, dass die Träger innerhalb der Schiene ungewollt verschoben werden. Alternativ kann hierzu eine Raste oder eine Sperre eingesetzt werden. In einer weiteren Ausführungsform weist jeder Bleigummilappen eine Öse mit einem Durchgangsloch auf, an der der Bleigummilappen von einer Schwenkachse gehalten wird.

Die US 5 006 718 A offenbart eine Strahlenschutzanordnung, mit 2 voneinander beabstandeten U-Profilen. Bleigumilappen sind verschwenkbar an einem Halter aufgehängt, der auf die Trägerschiene eines Tisches aufsetzbar ist und eine Befestigungseinrichtung aufweist, mit der der Halter an der Trägerschiene befestigbar ist. Die Befestigungseinrichtung weist ein erstes und zweites Klammerteil auf.

Der Erfindung liegt die Aufgabe zugrunde, eine Strahlenschutzanordnung bereitzustellen, die einfach an einem Behandlungstisch anzubringen und einfach handhabbar ist. Desweiteren ist es eine Aufgabe der Erfindung eine Strahlenschutzanordnung bereitzustellen, die in einfacher Art und Weise mit weiteren Strahlenschutzanordnungen verbunden werden kann und wobei auch in Schräglage des Behandlungstisches die Strahlenschutzanordnungen in einer vordefinierten Position zueinander verharren.

Diese Aufgaben werden mit einer Strahlenschutzanordnung gemäß den Patentansprüchen gelöst.

Die Erfindung geht dabei von dem Grundgedanken aus, eine Strahlenschutzanordnung mit einem zugehörigen Halter und einer entsprechenden Befestigungseinrichtung durch eine entsprechende Schwenkbewegung an einer Trägerschiene des Behandlungstisches zu befestigen und somit sowohl eine einfache Anbringung der Strahlenschutzanordnungen an dem Behandlungstisch als auch einen festen Sitz im befestigten Zustand zu gewährleisten. Die Erfindung hat dabei den Vorteil, dass die feste Positionierung der Strahlenschutzanordnungen an der Trägerschiene schnell und sicher ausgeführt werden kann und die Strahlenschutzanordnung für die Positionierung nicht abgesetzt werden muss, sondern durch eine einfach auszuführende Schwenkbewegung sicher an dem Behandlungstisch bzw. einer Trägerschiene des Behandlungstisches befestigt werden kann.

Die Strahlenschutzanordnung kann mit einem Teil einer Gelenkverbindung bereitgestellt werden, an dem eine weitere Strahlenschutzanordnung mit einem weiteren Teil der Gelenkverbindung angebracht werden kann, so dass die beiden Teile der Gelenkverbindung eine gerasterte Gelenkverbindung bilden.

Durch die Rasterung der Gelenkverbindung sind die miteinander verbundenen Strahlenschutzanordnungen auch bei Schräglage des Behandlungstisches fest zueinander positioniert und auch die Veränderung der Position der Strahlenschutzanordnung durch unbeabsichtigtes Anstoßen einer oder mehrerer Strahlenschutzanordnungen kann durch die Rasterung der Gelenkverbindungen vermieden werden.

Die Erfindung betrifft eine Strahlenschutzanordnung, zur Anbringung an einer Trägerschiene, die an einer Seite eines Behandlungstisches angebracht ist, mit:
einem Halter, an dem ein Strahlenschutzvorhang angeordnet ist, wobei der Halter auf die Trägerschiene aufsetzbar ist und eine Befestigungseinrichtung aufweist, mit der der Halter an der Trägerschiene befestigbar ist. Die Befestigungseinrichtung ist an einer Seite des Halters ausgebildet ist und weist mindestens ein erstes Klammerteil und mindestens ein zweites Klammerteil auf. Das erste und das zweite Klammerteil weisen jeweils ein L-Profil mit einem ersten Schenkel und einem zweiten Schenkel auf,
wobei das erste und das zweite Klammerteil derart angeordnet sind, dass die ersten Schenkel parallel ausgerichtet sind und die zweiten Schenkel zueinander zeigen. Das erste Klammerteil ist an einem oberen Rand des Halters und das zweite Klammerteil ist an einem unteren Rand des Halters angeordnet. Dadurch ist das erste Klammerteil bezogen auf eine Längsrichtung des Halters derart in einem ersten Abstand zu dem zweiten Klammerteil angeordnet, dass der Halter in einer ersten Position, in der der Halter gegenüber der Trägerschiene verschwenkt ist, mit der Trägerschiene in Kontakt bringbar ist. Durch eine Schwenkbewegung ist der Halter in eine zweite Position bringbar ist, in der der Halter auf der Trägerschiene befestigt ist.

Gemäß einer erfindungsgemäßen Ausführungsform weist die Befestigungseinrichtung ein drittes Klammerteil auf. Vorzugsweise ist das dritte Klammerteil in Längsrichtung des Halters in einem zweiten Abstand zu dem zweiten Klammerteil angeordnet. Vorzugsweise ist der zweite Abstand n-mal größer als der erste Abstand und besonders bevorzugt liegt n im Bereich von 1,5 bis 30.

Gemäß einer erfindungsgemäßen Ausführungsform weist das dritte Klammerteil ein L-Profil und/oder eine Fixiereinrichtung aufweist.

Gemäß einer erfindungsgemäßen Ausführungsform weist die Befestigungseinrichtung ein viertes Klammerteil auf. Vorzugsweise ist das vierte Klammerteil in Längsrichtung des Halters in einem dritten Abstand zu dem dritten Klammerteil angeordnet. Vorzugsweise ist der dritte Abstand m-mal größer als der erste Abstand und besonders bevorzugt liegt m im Bereich von 0,5 bis 15.

Gemäß einer erfindungsgemäßen Ausführungsform weist das vierte Klammerteil ein L-Profil und/oder eine Fixiereinrichtung auf.

Gemäß einer erfindungsgemäßen Ausführungsform weisen die L-Profile des dritten und des vierten Klammerteils - sofern das dritte und das vierte Klammerteil jeweils ein L-Profil aufweisen - jeweils einen ersten Schenkel und einen zweiten Schenkel auf, wobei das dritte und das vierte Klammerteil derart angeordnet sind, dass die ersten Schenkel des dritten und vierten Klammerteils parallel ausgerichtet sind und die zweiten Schenkel des dritten und vierten Klammerteils in die gleiche Richtung zeigen und das dritte und vierte Klammerteil derart angeordnet sind, dass die ersten Schenkel des dritten und vierten Klammerteils im aufgesetzten Zustand auf der Trägerschiene aufliegen.

Vorzugsweise kann sich an dem ersten Schenkel mindestens eines Klammerteils ein Auflagepuffer befinden, der so angeordnet ist, dass er im befestigten Zustand der Strahlenschutzanordnung an dem Behandlungstisch auf der Trägerschiene des Behandlungstisches aufliegt. Vorzugsweise enthält der Auflagepuffer mindestens einen der folgenden Materialien: Gummi, Kunststoff, Polyamid, PTFE, PBT, Polybutylenterephthalat, Polyphenylensulfid, Aramid.

Gemäß einer erfindungsgemäßen Ausführungsform weist der Halter ferner eine Zusatzschiene auf, die an der der Befestigungseinrichtung abgewandten Seite angebracht ist.

Die Erfindung wird nachstehend anhand von Beispielen und der Zeichnung näher erläutert. Es zeigen:
Figur 1 eine perspektivische Ansicht einer bevorzugten Ausführungsform einer Strahlenschutzanordnung gemäß der vorliegenden Erfindung,
Figur 2 eine perspektivische Ansicht eines Beispiels einer Strahlenschutzanordnung,
Figur 3 eine Seitenansicht einer Strahlenschutzanordnung,
Fig. 4 eine perspektivische Ansicht eines Beispiels einer Strahlenschutzanordnung,
Fig. 5 eine perspektivische Ansicht einer Strahlenschutzanordnung,
Fig. 6 eine perspektivische Ansicht einer Strahlenschutzanordnung,
Fig. 7 eine perspektivische Ansicht einer Strahlenschutzanordnung,
Fig. 8 eine perspektivische Ansicht einer Strahlenschutzanordnung, und
Fig. 9 eine perspektivische Ansicht einer Strahlenschutzanordnung.

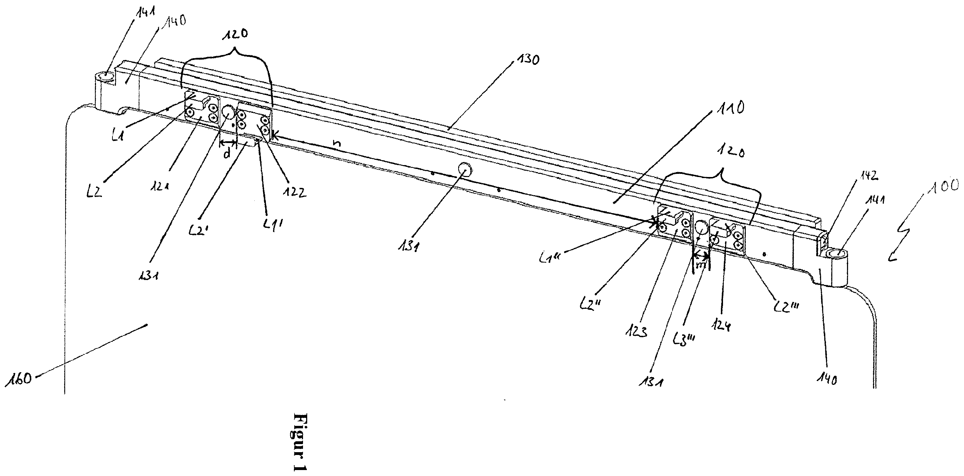

Figur 1 zeigt eine Ausführungsform einer Strahlenschutzanordnung 100 gemäß der vorliegenden Erfindung zur Anbringung an einen Behandlungstisch. Die Strahlenschutzanordnung 100 weist einen Halter 110 auf, der auf eine Trägerschiene eines Behandlungstisches (nicht gezeigt) aufgesetzt werden kann. An dem Halter 110 ist ein Strahlenschutzvorhang 160 angebracht. Ferner weist der Halter 110 eine Befestigungseinrichtung 120 auf. Gemäß der in Figur 1 gezeigten bevorzugten Ausführungsform der vorliegenden Erfindung weist die Befestigungseinrichtung 120 ein erstes Klammerteil 121, ein zweites Klammerteil 122, ein drittes Klammerteil 123 sowie ein viertes Klammerteil 124 auf. Die Klammerteile 121, 122, 123, 124 sind dabei an einer langen Seite des Halters 110 befestigt und zeigen im aufgesetzten Zustand in Richtung des Behandlungstisches. Die Klammerteile 121, 122, 123, 124 weisen jeweils ein L-Profil mit einem ersten Schenkel L1, L1', L1", L1'" und einem zweiten Schenkel L2, L2', L2", L2'" auf. Wie anhand von Figur 1 ersichtlich, sind die ersten Schenkel L1, L1' des ersten Klammerteils 121 und des zweiten Klammerteils 122 parallel zueinander ausgerichtet und die zweiten Schenkel L2, L2' des ersten Klammerteils 121 und des zweiten Klammerteils 122 zeigen zueinander. Das erste Klammerteil 121 ist an einem oberen Rand des Halters 110 und das zweiteKlammerteil 122 an einem unteren Rand des Halters 110 angeordnet. Das erste Klammerteil 121 ist bezogen auf eine Längsrichtung des Halters 110 derart in einem ersten Abstand d zu dem zweiten Klammerteil 122 angeordnet, dass der Halter 110 in einer ersten Position, in der der Halter 110 gegenüber der der Trägerschiene in einem Winkel verschwenkt ist, mit der Trägerschiene in Kontakt bringbar ist und durch eine Schwenkbewegung in eine zweite Position bringbar ist, in der der Halter 110 auf der Trägerschiene befestigt ist.

Gemäß der in Figur 1 gezeigten bevorzugten Ausführungsform der vorliegenden Erfindung sind das dritte Klammerteil 123 und das vierte Klammerteil 124 derart angeordnet, dass die ersten Schenkel L1", L1"' des dritten und vierten Klammerteils 123,124 parallel zueinander ausgerichtet sind und die zweiten Schenkel L2", L2'" des dritten und vierten Klammerteils 123, 124 in die gleiche Richtung zeigen und das dritte und vierte Klammerteil 123, 124 derart angeordnet sind, dass die ersten Schenkel L1", L1'" im aufgesetzten Zustand auf der Trägerschiene aufliegen.

Der Abstand n des dritten Klammerteils 123 zu dem zweiten Klammerteil 122 ist in der vorliegenden Ausführungsform 25-mal größer als der 1. Abstand d zwischen dem ersten Klammerteil 121 und dem zweiten Klammerteil 122. Der Abstand des vierten Klammerteils 124 zu dem dritten Klammerteil 123 ist in der vorliegenden Ausführungsform genauso groß wie der erste Abstand d zwischen dem ersten Klammerteil 121 und dem zweiten Klammerteil 122.

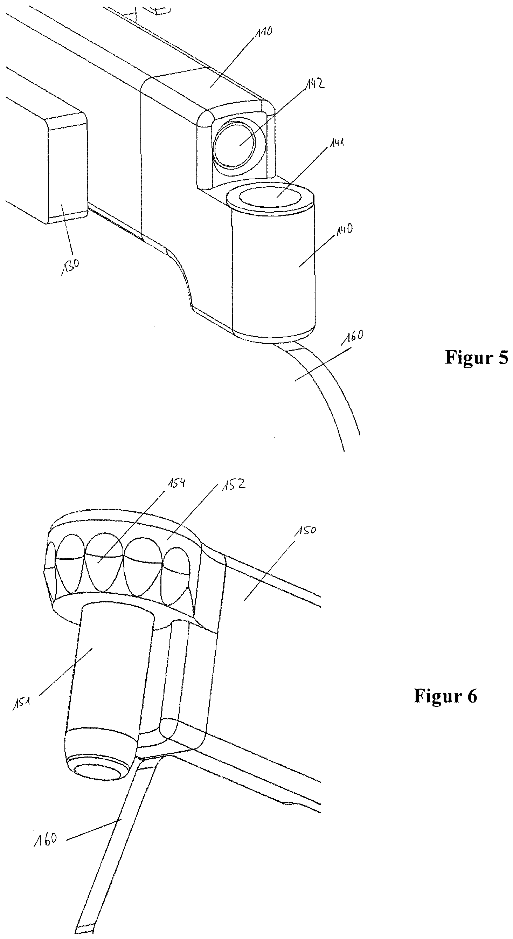

Der Halter 110 weist an seinen beiden kurzen Seiten jeweils ein erstes Teil 140 einer Gelenkverbindung auf. Der erste Teil 140 der Gelenkverbindung weist eine Bohrung 141 sowie eine Andrückeinrichtung 142 auf. Der erste Teil 140 der Gelenkverbindung ist geeignet mit einem zweiten Teil 150 der Gelenkverbindung, die beispielsweise an einem Halter einer zweiten Strahlenschutzanordnung befestigt ist, derart in Verbindung gebracht zu werden, dass der erste und der zweite Teil 140, 150 der Gelenkverbindung eine gerasterte Gelenkverbindung bilden. Der erste Teil 140 weist eine Bohrung 141 sowie eine Andrückeinrichtung 142 auf. Die ersten und zweiten Teile 140, 150 der Gelenkverbindung werden unten in den Figuren 5 bis 8 eingehend erörtert.

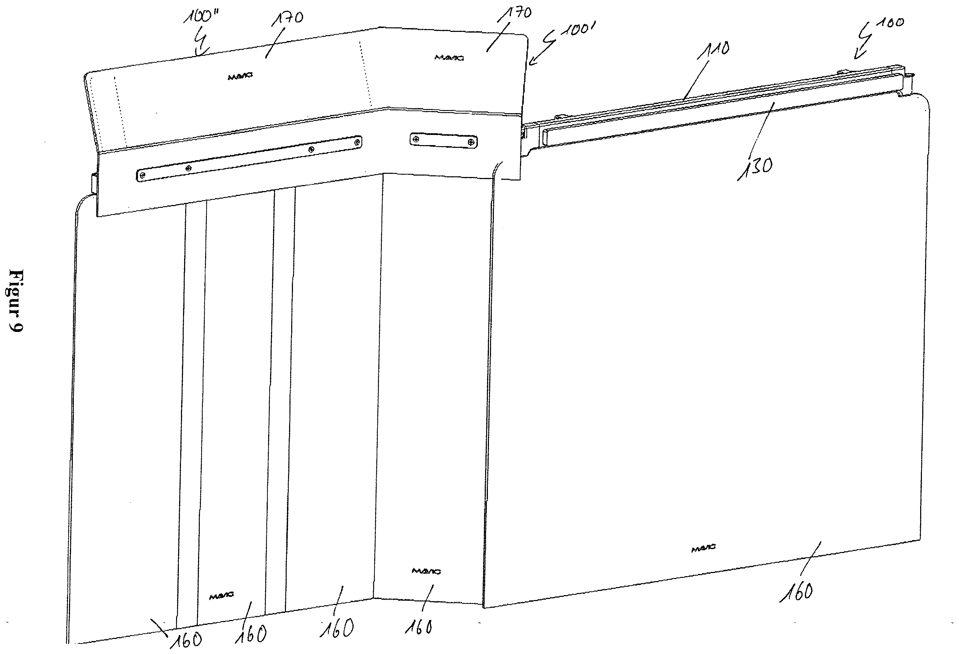

Des Weiteren weist der Halter 110 eine Zusatzschiene 130 auf, die an der der Befestigungseinrichtung 120 abgewandten Seite angebracht ist. Die Zusatzschiene 130 wird dabei durch entsprechende Bolzen 131 mit dem Halter 110 verbunden, so dass diese bevorzugt von dem Halter 110 etwas beanstandet ist. Diese Beanstandung der Zusatzschiene 130 von dem Halter 110 erleichtert das etwaige Aufsetzen von zusätzlichen Strahlenschutzschilden, wie in Figur 9 gezeigt.

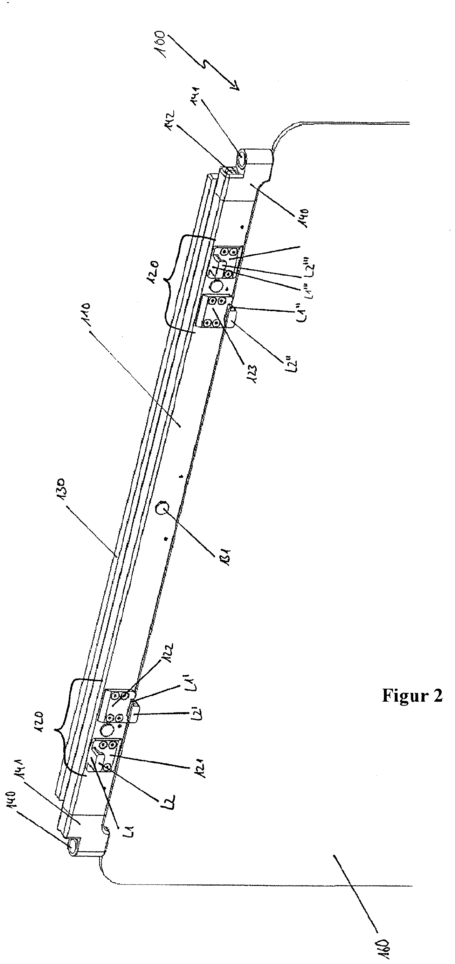

Figur 2 zeigt ein Beispiel einer Strahlenschutzanordnung 100 zur Anbringung an einen Behandlungstisch. Die Strahlenschutzanordnung 100 weist ebenso wie die in Figur 1 gezeigte Ausführungsform einen Halter 110 auf, der auf eine Trägerschiene eines Behandlungstisches aufgesetzt werden kann. Das in Figur 2 gezeigte Beispiel unterscheidet sich von der in Figur 1 gezeigten Ausführungsform dadurch, dass die zweiten Schenkel L2", L2'" des dritten und vierten Klammerteils 123, 124 ebenso wie die zweiten Schenkel L2, L2' des ersten Klammerteils 121 und des zweiten Klammerteils 122 zueinander zeigen. Dabei wird die Strahlenschutzanordnung 100 bzw. der Halter 110 seitlich auf die Trägerschiene aufgeschoben, ohne dass der Halter 110 bezüglich der Trägerschiene des Behandlungstisches verschwenkt wird.

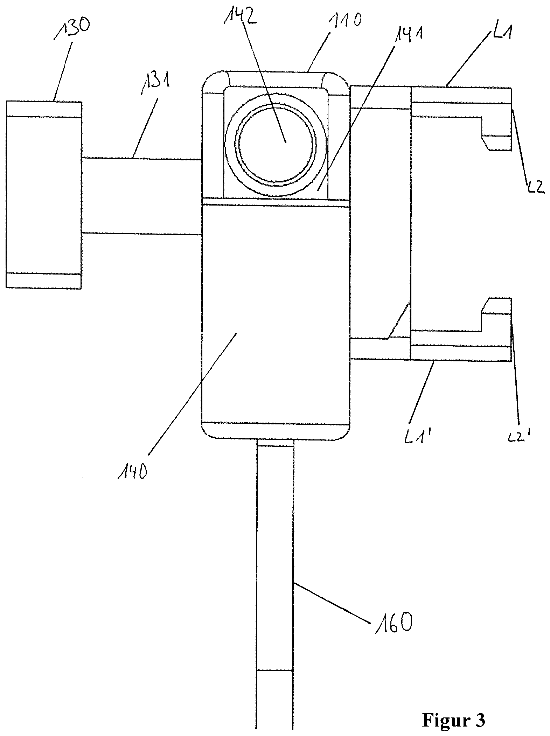

Figur 3 zeigt eine Seitenansicht eines Halters 110. Die in Figur 3 gezeigte Ausführungsform entspricht dabei in ihrer Seitenansicht sowohl der in Figur 1 gezeigten Ausführungsform als auch der in Figur 2 gezeigten Ausführungsform.

So weist der Halter 110 an seiner dem Behandlungstisch zugewandten langen Seite die Befestigungseinrichtung 120 mit dem ersten und dem zweiten Klammerteil 121, 122 auf, wobei die beiden Klammerteile 121, 122 jeweils ein L-Profil aufweisen mit den bereits diskutierten ersten und zweiten Schenkeln L1, L1', L2, L2'. An der Stirnseite bzw. der kurzen Seite des Halters 110 befindet sich der erste Teil 140 der Gelenkverbindung, der die Bohrung 141 und die Andrückeinrichtung 142 aufweist. An der dem Behandlungstisch abgewandten langen Seite weist der Halter 110 Befestigungsbolzen 131 auf, an denen die Zusatzschiene 130 befestigt sind. Durch die Länge der Bolzens 131 ist dabei die Zusatzschiene 130 von dem Halter 110 beanstandet.

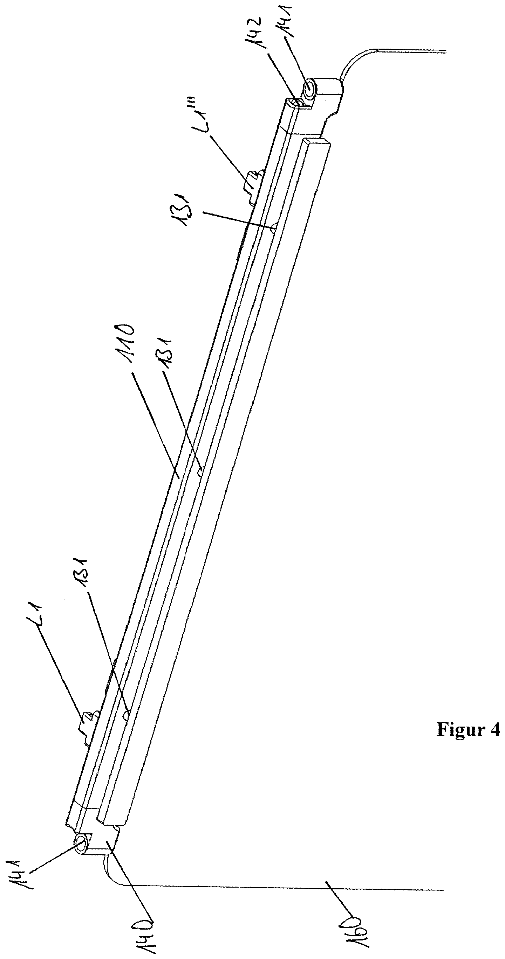

Figur 4 zeigt ein Beispiel einer Strahlenschutzanordnung 100 zur Anbringung an einen Behandlungstisch. Das Beispiel gleicht dem in Figur 2 gezeigten Beispiel. Insbesondere ist jedoch in Figur 4 noch einmal die Zusatzschiene 130 vollständig gezeigt.

Figur 5 zeigt eine Ausführungsform des ersten Teils 140 einer Gelenkverbindung. Der erste Teil 140 der Gelenkverbindung ist an der kurzen Seite des Halters 110 befestigt. Ebenso sind die Zusatzschiene 130 sowie der Schutzvorhang 160 an dem Halter 110 befestigt. Gezeigt ist ferner die Andrückeinrichtung 142, die gemäß der vorliegenden Ausführungsform eine Kugeldruckschraube aufweist. Des Weiteren weist der erste Teil 140 der Gelenkverbindung die Bohrung 141 auf. Gemäß der vorliegenden Ausführungsform befindet sich in der Bohrung eine Gleitlagerbuchse aus Kunststoff, bevorzugt PTFE.

Figur 6 zeigt eine Ausführungsform des zweiten Teils 150 einer Gelenkverbindung. Ebenso wie der erste Teil 140 der Gelenkverbindung kann auch der zweite Teil 150 der Gelenkverbindung an der kurzen Seite des Halters 110 befestigt werden. Ebenso kann der zweite Teil 150 der Gelenkverbindung an einem zusätzlichen Strahlenschutzschild ohne Halter 110 befestigt werden, so dass die zusätzliche Strahlenschutzschild bzw. die zusätzliche Strahlenschutzanordnung nicht direkt mit dem Behandlungstisch in Kontakt ist. Der zweite Teil 150 der Gelenkverbindung weist einen Kopf 152 sowie einen Bolzen 151 auf. Der Kopf 152 weist mehrere Aussparungen 154 auf. Der Bolzen 151 des zweitens Teils 150 der Gelenkverbindung kann in die Bohrung 141 des ersten Teils 140 der Gelenkverbindung gesteckt werden. Die Aussparungen 154 des Kopfes 152 des zweiten Teils 150 der Gelenkverbindung sind derart ausgestaltet, dass durch das Steckens des Bolzens in die Bohrung 141 die Andrückeinrichtung 142 von der Aussparung 154 aufgenommen wird. Vorliegend weisen die Aussparungen 154 deshalb an der dem Bolzen 151 zugewandten Seite passende Gänge auf, so dass beim Einsetzen des Bolzens 151 in die Bohrung 141 die Andrückeinrichtung 142 leicht von den Aussparungen 154 aufgenommen werden kann.

Des Weiteren weist der Bolzen 151 an seinem dem Kopf 152 abgewandten Ende eine Fasung auf, die das Einführen des Bolzens 151 in die Bohrung 141 erleichtert.

Figur 6 zeigt eine weitere Ausführungsform des zweiten Teils 150 einer Gelenkverbindung. Zwischen dem Bolzen 151 und dem Hauptkörper des zweiten Teils 150 der Gelenkverbindung liegt ein Winkel von 0,75°. Anders ausgedrückt steht der Bolzen 151 nicht parallel zum Hauptkörper des 2. Teils 150 der Gelenkverbindung. Dieser Winkel, bevorzugt 0,75°, sorgt für eine Vorspannung am Bolzen. Diese Vorspannung sorgt dafür, dass bei Belastung der Gelenkverbindung, beispielsweise durch ein freistehendes Ende an einem Teil 140, 150 der Gelenkverbindung die beiden durch die Gelenkverbindung verbundenen Strahlenschutzanordnungen miteinander bündig abschließen.

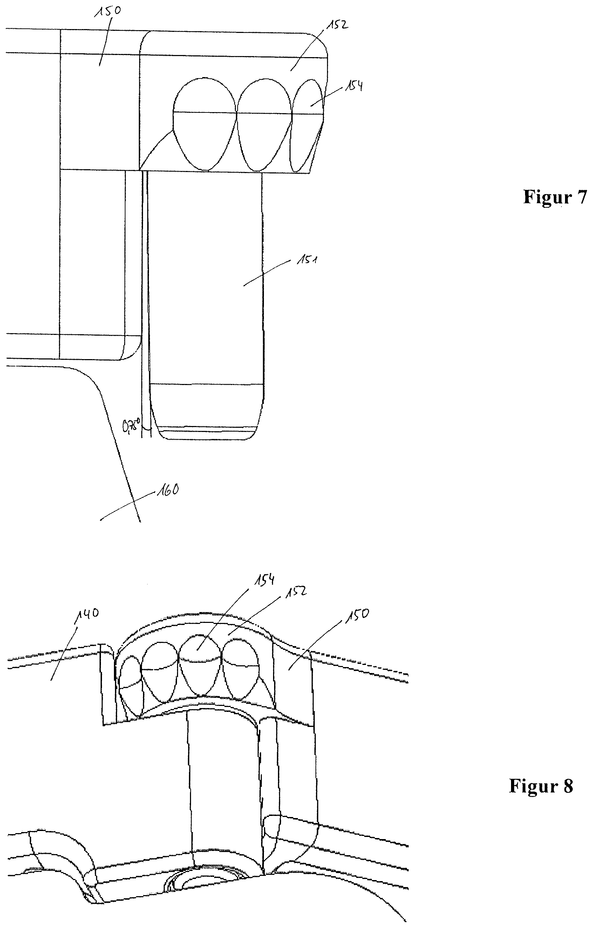

Figur 8 zeigt eine zusammengesetzte Gelenkverbindung aus einem ersten Teil 140 und einem zweiten Teil 150. Es ist deutlich zu erkennen, dass die Andrückeinrichtung 142 von einer Aussparung 154 aufgenommen wurde, während die anderen Aussparungen 154 des Kopfes 152 nicht besetzt sind und die Aussparungen 154 insgesamt ein Raster bilden, so dass durch die gerasterte Gelenkverbindung vordefinierte Winkeleinstellungen eingenommen werden können.

Durch Aufwendung einer vordefinierten Kraft, die auf den ersten Teil 140 ausgeübt wird, kann die Andrückeinrichtung 142 von einer ersten Rastposition, in der die Andrückeinrichtung 142 sich in einer ersten Aussparung 154 befindet, in eine zweite Rastposition in der die Andrückeinrichtung 142 sich in einer zweiten Aussparung 154 befindet, bewegt werden. Anders gesagt, kann durch Verdrehen der Gelenkverbindung gegen eine vordefinierte Kraft die Andrückeinrichtung 142 von einer ersten in eine zweite Rastposition bewegt werden.

Figur 9 zeigt ein zusammengesetztes System aus mehreren Strahlenschutzanordnungen 100, 100', 100", die durch die in den Figuren 5 bis 8 beschriebenen Gelenkverbindungen miteinander verbunden sind.

Lediglich die rechte Strahlenschutzanordnung 100 weist einen Halter 110 auf und wird an dem Behandlungstisch befestigt, die Strahlenschutzanordnungen 100', 100", sind mit der Strahlenschutzanordnung 100 verbunden, ohne mit dem Behandlungstisch direkt verbunden zu sein. Des Weiteren weisen die Strahlenschutzanordnungen 100',100" steckbare Strahlenschutzschilde 170 auf, die an entsprechenden Schienen 130 befestigt sind. Durch die gerasterte Gelenkverbindungen ist es möglich die Positionen der Strahlenschutzanordnungen 100, 100', 100" in einen durch die Aussparungen 154 vordefinierten Winkel zu einander festzustellen. Somit können die Positionen der Strahlenschutzanordnungen 100, 100', 100" zueinander sowohl bei Schräglage des Behandlungstisches gehalten werden als auch bei unbeabsichtigten Anstoßens der Strahlenschutzanordnungen 100, 100', 100" bspw. durch das Bedienpersonal.

## Patentansprüche

1. Strahlenschutzanordnung zur Anbringung an einer Trägerschiene, die an einer Seite eines Behandlungstisches angebracht ist, mit:
einem Halter, an dem ein Strahlenschutzvorhang angeordnet ist, wobei der Halter auf die Trägerschiene aufsetzbar ist und eine Befestigungseinrichtung aufweist, mit der der Halter an der Trägerschiene befestigbar ist,
wobei die Befestigungseinrichtung an einer langen Seite des Halters ausgebildet ist und im aufgesetzten Zustand in Richtung des Behandlungstisches zeigt, und
mindestens ein erstes Klammerteil und mindestens ein zweites Klammerteil aufweist, wobei das erste und das zweite Klammerteil jeweils ein L-Profil mit einem ersten Schenkel (L1, L1') und einem zweiten Schenkel (L2, L2') aufweisen,
wobei das erste und das zweite Klammerteil derart angeordnet sind, dass die ersten Schenkel (L1, L1') parallel ausgerichtet sind und die zweiten Schenkel (L2, L2') zueinander zeigen, und wobei das erste Klammerteil an einem oberen Rand des Halters und das zweite Klammerteil an einem unteren Rand des Halters angeordnet sind, dass das erste Klammerteil bezogen auf eine Längsrichtung des Halters derart in einem ersten Abstand zu dem zweiten Klammerteil angeordnet ist, dass der Halter in einer ersten Position, in der der Halter gegenüber der Trägerschiene verschwenkt ist, mit der Trägerschiene in Kontakt bringbar ist und durch eine Schwenkbewegung in eine zweite Position bringbar ist, in der der Halter auf der Trägerschiene befestigt ist.

2. Strahlenschutzanordnung nach Anspruch 1, wobei die Befestigungseinrichtung ein drittes Klammerteil aufweist, das vorzugsweise in Längsrichtung des Halters in einem zweiten Abstand zu dem zweiten Klammerteil angeordnet ist, wobei der zweite Abstand vorzugsweise n-mal größer als der erste Abstand ist und besonders bevorzugt n im Bereich von 1,5 bis 15 liegt.

3. Strahlenschutzanordnung nach Anspruch 2, wobei das dritte Klammerteil ein L-Profil und/oder eine Fixiereinrichtung aufweist.

4. Strahlenschutzanordnung nach Anspruch 2 oder 3, wobei die Befestigungseinrichtung ein viertes Klammerteil aufweist, das vorzugsweise in Längsrichtung des Halters in einem dritten Abstand zu dem dritten Klammerteil angeordnet ist, wobei der dritte Abstand vorzugsweise m-mal größer als der erste Abstand ist und besonders bevorzugt m im Bereich von 0,5 bis 15 liegt.

5. Strahlenschutzanordnung nach Anspruch 4, wobei das vierte Klammerteil ein L-Profil und/oder eine Fixiereinrichtung aufweist.

6. Strahlenschutzanordnung nach Anspruch 5, sofern das dritte und das vierte Klammerteil jeweils ein L-Profil aufweisen, wobei die L-Profile des dritten und des vierten Klammerteils jeweils einen ersten Schenkel (L1", L1"') und einen zweiten Schenkel (L2", L2'") aufweisen, wobei das dritte und das vierte Klammerteil derart angeordnet sind, dass die ersten Schenkel (L1", L1"') parallel ausgerichtet sind und die zweiten Schenkel (L2', L2'") in die gleiche Richtung zeigen und das dritte und vierte Klammerteil derart angeordnet sind, dass die ersten Schenkel (L1", L1"') des dritten und vierten Klammerteils im aufgesetzten Zustand auf der Trägerschiene aufliegen.

7. Strahlenschutzanordnung nach einem der vorstehenden Ansprüche, wobei der Halter ferner eine Zusatzschiene aufweist, die an der der Befestigungseinrichtung abgewandten Seite angebracht ist.

## Claims

1. A radiation protection arrangement for attachment to a support rail, which is attached to a side of a treatment table, comprising:
a holder on which a radiation protection drape is arranged, the holder being attachable to the support rail and comprising a fastening means with which the holder can be fastened to the support rail,
wherein the fastening means is formed on a long side of the holder and in the mounted state points in the direction of the treatment table, and comprises at least one first bracket part and at least one second bracket part,
wherein each of the first and second bracket parts has an L-shaped profile with a first leg (L1, L1') and a second leg (L2, L2'),
wherein the first and the second bracket parts are arranged such that the first legs (L1, L1') are aligned in parallel and the second legs (L2, L2') face each other, and wherein the first bracket part is arranged at an upper edge of the holder and the second bracket part is arranged at a lower edge of the holder, that the first bracket part is arranged relative to a longitudinal direction of the holder at a first distance from the second bracket part such that the holder in a first position in which the holder is pivoted relative to the support rail can be brought into contact with the support rail and, with a pivoting motion, can be brought into a second position in which the holder is attached to the support rail.

2. The radiation protection arrangement according to claim 1, wherein the fastening means comprises a third bracket part which is preferably arranged in a longitudinal direction of the holder at a second distance from the second bracket part, wherein the second distance is preferably n times greater than the first distance and n is particularly preferably in the range of from 1.5 to 15.

3. The radiation protection arrangement according to claim 2, wherein the third bracket part has an L-shaped profile and/or comprises a fixing device.

4. The radiation protection arrangement according to claim 2 or 3, wherein the fastening means comprises a fourth bracket part which is preferably arranged in longitudinal direction of the holder at a third distance from the third bracket part, wherein the third distance is preferably m times greater than the first distance and m is particularly preferably in the range of from 0.5 to 15.

5. The radiation protection arrangement according to claim 4, wherein the fourth bracket part has an L-shaped profile and/or comprises a fixing device.

6. The radiation protection arrangement according to claim 5, with the proviso that the third and fourth bracket parts each have an L-shaped profile, wherein the L-shaped profiles of the third and fourth bracket parts each have a first leg (L1", L1'") and a second leg (L2", L2"'), wherein the third and fourth bracket parts are arranged such that the first legs (L1", L1"') are aligned in parallel and the second legs (L2', L2"') show in the same direction and the third and fourth bracket parts are arranged such that, in the fitted state, the first legs (L1", L1"') of the third and fourth bracket parts rest on the support rail.

7. The radiation protection arrangement according to any one of the preceding claims,
wherein the holder further comprises an additional rail which is attached to the side opposite to the fastening means.

## Revendications

1. Système de protection contre les rayonnements, mis en place sur un rail porteur monté sur un côté d'une table de traitement, comprenant :
un support, sur lequel est disposé un rideau de protection contre les rayonnements, ledit support pouvant être monté sur le rail porteur et présentant un dispositif de fixation au moyen duquel le support peut être fixé sur le rail porteur,
où le dispositif de fixation est prévu sur un côté long du support et dirigée vers la table de traitement en état de montage , et
comprend au moins une première partie de pince et au moins une deuxième partie de pince, la première et la deuxième partie de pince comportant chacune un profilé en L avec une première aile (L1, L1') et une deuxième aile (L2, L2'),
où la première et la deuxième partie de pince sont disposées de telle manière que les premières ailes (L1, L1') sont orientées parallèlement et que les deuxièmes ailes (L2, L2') sont dirigées l'une vers l'autre, la première partie de pince étant disposée sur un bord supérieur du support et la deuxième partie de pince sur un bord inférieur du support, et que la première partie de pince est disposée à un premier intervalle de la deuxième partie de pince dans le sens de la longueur du support, de manière à pouvoir mettre le support en contact avec le rail porteur dans une première position où le support est pivoté par rapport au rail porteur, et à pouvoir amener le support par pivotement vers une deuxième position où le support est fixé sur le rail porteur.

2. Système de protection contre les rayonnements selon la revendication 1, où le dispositif de fixation comprend une troisième partie de pince, avantageusement disposée à un deuxième intervalle de la deuxième partie de pince dans le sens de la longueur du support, ledit deuxième intervalle étant avantageusement n fois plus grand que le premier intervalle et n étant préférentiellement compris entre 1,5 et 15.

3. Système de protection contre les rayonnements selon la revendication 2, où la troisième partie de pince comporte un profilé en L et/ou un dispositif de fixation.

4. Système de protection contre les rayonnements selon la revendication 2 ou la revendication 3, où le dispositif de fixation comprend une quatrième partie de pince, avantageusement disposée à un troisième intervalle de la troisième partie de pince dans le sens de la longueur du support, ledit troisième intervalle étant avantageusement m fois plus grand que le premier intervalle et m étant préférentiellement compris entre 0,5 et 15.

5. Système de protection contre les rayonnements selon la revendication 4, où la quatrième partie de pince comporte un profilé en L et/ou un dispositif de fixation.

6. Système de protection contre les rayonnements selon la revendication 5, si la troisième et la quatrième partie de pince comprennent chacune un profilé en L, où les profilés en L de la troisième et de la quatrième partie de pince comportent chacune une première aile (L1", L1"') et une deuxième aile (L2", L2'"), où la troisième et la quatrième partie de pince sont disposées de telle manière que les premières ailes (L1", L1"') sont orientées parallèlement et que les deuxièmes ailes (L2', L2"') sont dirigées dans la même direction, et où la troisième et la quatrième partie de pince sont disposées de telle manière que les premières ailes (L1", L1'") de la troisième et de la quatrième partie de pince reposent sur le rail porteur en état de montage.

7. Système de protection contre les rayonnements selon l'une des revendications précédentes, où le support comprend également un rail additionnel monté sur le côté distant du dispositif de fixation.
